# FASCICULE DE BREVET EUROPEEN

(11) **EP 3 723 719 B1**
(45) Date de publication et mention de la délivrance du brevet: **20.10.2021**
(21) Numéro de dépôt: 18826416.2
(22) Date de dépôt: 27.11.2018
(51) Int. Cl.: A61K 8/60, A61K 9/00, A61K 31/7032, A61P 17/00, A61Q 19/00, C07H 15/04

(54) **NOUVEAUX GLYCÉRYL POLYRHAMNOSIDES, PROCÉDÉ POUR LEUR PRÉPARATION ET COMPOSITION COSMÉTIQUES ET/OU PHARMACEUTIQUES EN COMPRENANT**
NEUE GLYCERYLPOLYRHAMNOSIDE, VERFAHREN ZUR HERSTELLUNG DAVON UND KOSMETISCHE UND/ODER PHARMAZEUTISCHE ZUSAMMENSETZUNGEN DAMIT
NEW GLYCERYL POLYRHAMNOSIDES, PROCESS FOR THE PREPARATION THEREOF, AND COSMETIC AND/OR PHARMACEUTICAL COMPOSITION COMPRISING SAME

(30) Priorité: 12.12.2017 FR 1761986
(43) Date de publication de la demande: 21.10.2020
(73) Titulaire: Société d'Exploitation de Produits pour les Industries Chimiques SEPPIC, 75321 Paris cedex 07 (FR)
(72) Inventeur: CATTUZZATO, Laetitia, 81100 CASTRES (FR); DACOSTA, Georges, 81710 SAIX (FR); DESSILLA, Stéphane, 81100 CASTRES (FR); GUILBOT, Jérôme, 81100 CASTRES (FR)
(74) Mandataire: Air Liquide
(86) Numéro de dépôt international: PCT/FR2018/053010
(87) Numéro de publication internationale: WO 2019/115904

(56) Documents cités:
- DE-A1-102011 081 436
- FR-A1- 2 876 283
- FR-A1- 3 050 935
- JP-A- 2014 058 472

## Description

La présente invention a pour objet une nouvelle composition à base de dérivés de sucre, le procédé pour sa préparation et son utilisation dans les compositions cosmétiques et pharmaceutiques.

L'invention trouve principalement application en cosmétique et en pharmacie.

La peau est un organe atypique du corps humain, extrêmement fin au regard de son étendue mais également l'organe le plus lourd d'un individu. Une des caractéristiques de la peau réside dans le fait qu'il s'agit d'un organe d'interface, d'un organe limite, entre le milieu intérieur (corps humain) et le milieu extérieur. De ce fait, et avec la flore qui la recouvre et l'habite, la peau est la première barrière de protection de l'organisme humain. En raison de sa position d'interface avec le milieu extérieur, la peau est soumise à des sollicitations quotidiennes nombreuses, telles que par exemple le contact avec des vêtements, les changements de températures, les changements de degrés d'hygrométrie, les changements de pression, voire à des agressions, comme par exemple le contact avec certains produits chimiques présentant ou pouvant présenter un caractère très acide, ou très basique, ou irritant, avec des produits chimiques considérés comme des agents polluants. La peau est composée de couches de différents tissus :
- L'épiderme, composé de kératinocytes, est sa partie la plus externe, puis vient
- Le derme, qui est un tissu conjonctif composé principalement de fibroblastes et de la matrice extracellulaire, et
- L'hypoderme, constitué d'adipocytes, qui est la partie la plus profonde et la plus éloignée du milieu extérieur.

La peau assure diverses fonctions dans l'intérêt de l'ensemble du système qu'elle abrite parmi lesquelles on peut retenir :
- Une fonction de barrière mécanique pour garantir l'intégrité du milieu intérieur de l'organisme,
- Une fonction émonctorielle visant à sécréter de la sueur à base d'eau, de sels et de déchets acides,
- Une fonction de régulation de la température du corps, et contient bien d'autres mécanismes de régulation, comme par exemple son mécanisme d'adaptation et de protection face aux rayonnements ultra-violets (coloration pigmentaire adaptative par la production de la mélanine), comme par exemple un système de veille immunitaire par la présence de macrophages, de cellules dendritiques.

La peau humaine constitue aussi la première image offerte au regard d'autrui. Par conséquent, l'amélioration de son aspect est un sujet de préoccupation constant pour les êtres humains. La peau est le reflet d'un état de bien-être, souvent associé à la jeunesse, et a *contrario* d'un état de fatigue et/ou de vieillissement. Il en résulte que la préservation, l'amélioration de l'état de la couche la plus extérieure de la peau, à savoir l'épiderme, constitue un centre d'intérêt majeur pour les recherches menées par les industries cosmétiques.

A la périphérie de l'épiderme, se trouve une couche cornée supérieure, nommée le *stratum corneum,* qui est la première couche de l'épiderme à subir les stress d'origine externe, comme les variations de conditions climatiques extérieures (température, pression, hygrométrie) ou les sollicitations mécaniques. C'est pourquoi, l'amélioration de l'apparence ou le maintien de la bonne apparence de la peau humaine, consiste notamment à maintenir un état d'hydratation du *stratum corneum* à un niveau optimal et satisfaisant. Cela permet en plus d'éviter les inconvénients esthétiques et physiologiques liés à un état de sécheresse de la peau.

Le *stratum corneum* a tendance à se déshydrater et à sécher lorsqu'il est exposé à une faible humidité ou lorsqu'il est mis en contact de façon prolongée et fréquente avec une solution détergente.

De nombreuses solutions ont déjà été apportées pour résoudre les problèmes de sécheresse de la peau dues à une déshydratation du *stratum corneum,* notamment par la mise au point de compositions hydratantes, voir par exemple FR 3 050 935 A1, JP 2014 058472 A, DE 10 2011 081436 ou FR 2 876 283 A1.

Cependant, pour éviter que la peau ne se dégrade trop rapidement, il est nécessaire d'apporter une hydratation de la peau à court terme, agissant dans un bref délai, notamment dans le cas de l'exposition des mains, des lèvres et du visage à des conditions climatiques (froid intense, vent) induisant un rapide dessèchement de la peau.

Pour évaluer et rendre compte de la « bonne santé » de la peau, et de son « bon état fonctionnel », et plus particulièrement de « son hydratation », deux mesures sont couramment utilisées, pour leur fiabilité et leur robustesse :

La mesure du taux d'hydratation dans le *stratum corneum,* c'est-à-dire de la teneur en eau en son sein et la mesure de la perte insensible en eau (ou PIE), c'est-à-dire le flux d'eau s'évaporant de la peau par le *stratum corneum,* du fait de la diffusion passive d'eau des couches les plus profondes de la peau vers le milieu extérieur et du fait de la transpiration, pouvant être détecté à tout instant à sa surface.

Le taux d'hydratation de la peau est mesuré par des techniques bio-métrologiques, notamment fondée sur la les mesures de la capacitance cutanée ou l'impédance cutanée. Différents appareillages commerciaux sont disponibles pour effectuer de telles mesures. Une augmentation de l'impédance ou de la capacitance indique une augmentation du taux d'hydratation de la peau et par conséquent de la teneur en eau dans le *stratum corneum.*

La perte insensible en eau (ou PIE) est mesurée par des techniques bio-métrologiques consistant en l'application, à la surface de la peau et dans des conditions standardisées, d'un instrument constitué d'un cylindre ouvert dans lequel une sonde mesure, de façon dynamique, le flux d'évaporation d'eau. En complément de cette technique traditionnelle du cylindre ouvert, des instruments similaires, basés sur un système de cylindre fermé (« closed chamber »), ont été plus récemment développés et sont également disponibles. Une diminution de la « perte insensible en eau » mesurée à la surface du *stratum corneum* indique une réduction de l'évaporation de l'eau contenue

Au regard de ces précédentes définitions, une amélioration de l'hydratation de la peau, et plus particulièrement du *stratum corneum,* peut se concevoir par mise en contact de la peau avec des composés qui possèdent un « effet hydratant ».

Un enjeu de l'hydratation cutanée, et donc des recherches qui y sont associées, est de mettre au point des substances ou des compositions qui se caractérisent par un effet hydratant amélioré sans pour autant devoir induire un effet occlusif important, ledit effet occlusif pouvant provoquer des perturbations du fonctionnement naturel d'une peau « non pathologique », à savoir une peau qui ne présente pas une perte insensible en eau supérieure à 10g/m²/h et donc se révéler contraire au but recherché.

Comme agent hydratant de la peau, et plus particulièrement de peaux très sèches et déstructurées, il y a :
- Les agents occlusifs qui se caractérisent par leur aptitude à former un film imperméable à la surface de la peau et à diminuer ainsi fortement l'évaporation de l'eau à la surface de l'épiderme. De tels agents sont par exemple les huiles minérales comme la vaseline ou les paraffines liquides, la glycérine, le beurre de karité (Butyrospermum parkii butter), la cire d'abeille, certaines huiles végétales comme l'huile de germe de blé, l'huile de coco, e beurre de cacao, la lanoline et les dérivés de la silicone ;
- Les agents émollients qui se caractérisent par leur aptitude à combler les espaces intercellulaires se trouvant entre les cornéocytes (cellules de la couche cornée de l'épiderme) ; ils limitent aussi de l'évaporation de l'eau de l'épiderme, mais dans une moindre mesure que les agents occlusifs. De tels agents sont par exemple les céramides, l'acide linoléique et certaines huiles végétales comme huile d'amande douce ou huile de jojoba ;
- Les agents filmogènes, qui se caractérisent par leur aptitude à s'associer à l'eau pour former des hydrogels semi-perméables ; ils participent ainsi à la modulation de l'évaporation de l'eau du *stratum corneum.* De tels agents sont par exemple, le collagène, l'élastine, l'ADN, la pectine, la gélatine, le chitosan, ou les glycosaminoglycans comme l'acide hyaluronique ;
- Les agents humectants, substances hydrophiles et qui se caractérisent par leur fort pouvoir hygroscopique, à savoir leur aptitude à retenir l'eau. Ils contribuent ainsi à permettre au *stratum corneum* de conserver à la fois l'eau qu'il renferme, et celle apportée par la formule cosmétique. de tels agents sont par exemple l'urée, le glycérol, l'acide lactique, les acides aminés, le lactate de sodium, le propylène de glycol, les polyéthylèneglycols, les acides alpha-hydroxylés ou le sorbitol. Le glycérol, l'urée et l'acide lactique sont les agents humectants les plus utilisés dans les compositions cosmétiques hydratantes, et tout particulièrement le glycérol pour son coût très compétitif.

Cependant, certains agents humectants, comme la glycérine, ont aussi un effet occlusif immédiat, ce qui n'est pas désiré pour les peaux normales dont la fonction barrière n'est pas défaillante. De plus certains d'entre eux, comme aussi la glycérine, provoquent certaines irritations de la peau et des muqueuses chez des personnes particulièrement sensibles.

La recherche de nouvelles substances hydratantes, mieux tolérées que le glycérol, a notamment conduit à l'utilisation de certains de ses dérivés en particulier les acétals de glycérol, résultant de sa condensation avec un sucre réducteur. On peut citer notamment :
- Les acétals issus de la condensation du glycérol avec le glucose, qui sont divulgués dans la demande de brevet européen publiée sous le numéro EP 0 770 378 ; ils présentent effectivement une meilleure tolérance cutanée que le glycérol mais ont un pouvoir hydratant généralement plus faible.
- Le mannosyl érythritol décrit dans la demande de brevet japonais publiée sous le numéro JP 63063390 A2 qui est obtenu selon un procédé de préparation par voie enzymatique, et connu pour son activité de rétention de l'hydratation cutanée.

Parmi les acétals du glucose et d'autres polyols, on peut citer les glycosides obtenus par acétalisation de l'érythritol, du xylitol ou du sorbitol, tels que décrits dans la demande de brevet français publiée sous le numéro FR 2 839 447 A1. Ces composés ont démontré de meilleures propriétés hydratantes que les produits d'acétalisation du glycérol susmentionnés.

Les polyol polyglycosides (ou PPG) sont obtenus de la même manière que les APG à partir de glucose et en remplaçant les alcools gras de départ par des polyols. A la fin des réactions de glycosylation, les produits peuvent être commercialisés tels quels en solution dans le polyol de départ ou sous forme de solutions aqueuses. De manière similaire aux alkyl polyglucosides (ou APG), les PPG ciblés se caractérisent par un degré de polymérisation moyen (DP) très proche de 1. Seuls des polyol polyglucosides sont disponibles commercialement, à savoir un glycéryl polyglucosidé commercialisé sous le nom de marque Hydragen™GG et une composition comprenant du xylityl polyglucoside commercialisée sous le nom de marque Aquaxyl™.

Dans le cadre de leurs recherches concernant l'amélioration de l'hydratation de la peau, les inventeurs se sont attachés à développer une nouvelle solution technique fondée sur l'utilisation d'un sucre naturel jamais utilisé dans ce cadre, le rhamnose, dont la source naturelle est l'hémicellulose du bois, notamment celle du boulot ou du hêtre :

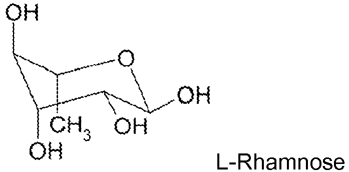

Ainsi, selon un premier aspect, l'invention a pour objet une composition (C₁) comprenant pour 100% de sa masse :
(1)- Une quantité supérieure à 0% en masse et inférieure ou égale à 50% en masse d'un polyol de formule (I) :

   HO-CH₂-CHOH-CH₂-OH (I) ;
(2)- De 25% en masse à 95% en masse d'une composition (C_{R}) représentée par la formule (II) :

   HO-CH₂-CHOH-CH₂-O-(Rham)ₓ-H (II),

   formule (II) dans laquelle Rham représente le reste du rhamnose et x, qui indique le degré moyen de polymérisation dudit reste Rham, représente un nombre décimal supérieur à 1,0 et inférieur ou égal à 2.5,
(3)- Une quantité supérieure à 0% en masse et inférieure ou égale à 20% en masse de rhamnose,

Par la formule (II) HO-CH₂-CHOH-CH₂-O-(Rham)ₓ-H, on signifie que ladite composition (C_{R}) consiste essentiellement en un mélange de composés représentés par les formules (II₁), (II₂), (II₃), (II₄) et (II₅) :

HO-CH₂-CHOH-CH₂-O-(Rham)₁-H (II₁),

HO-CH₂-CHOH-CH₂-O-(Rham)₂-H (II₂),

HO-CH₂-CHOH-CH₂-O-(Rham)₃-H (II₃),

HO-CH₂-CHOH-CH₂-O-(Rham)₄-H (II₄),

HO-CH₂-CHOH-CH₂-O-(Rham)₅-H (II₅),

dans les proportions molaires respectives a₁, a₂, a₃, a₄ et a₅, telles que la somme a₁+a₂+a₃+a₄+a₅ est égale à 1 et que la somme a₁+2a₂+3a₃+4a₄+5a₅ est égale à x.

Par essentiellement, on indique dans la définition qui précède, que la présence d'un ou de plusieurs composés de formule (II_{w}) avec w supérieur à 5 n'est pas exclue au sein de la composition (C_{R}), mais que si présence il y a, elle l'est en des proportions minimes qui n'entraînent aucune modification substantielle des propriétés de ladite composition (C_{R}).

Dans la formule (II) telle que définie ci-dessus, le groupe HO-CH₂-CHOH-CH₂-O- est lié à (Rham)ₓ par le carbone anomérique du reste saccharide, de manière à former une fonction acétal.

Selon un aspect particulier de la présente invention, x représente dans la formule (II), un nombre décimal supérieur ou égal à 1,2 et inférieur ou égal à 2,2.

Selon un autre aspect particulier, l'invention a pour objet la composition (C₁) telle que définie précédemment, comprenant pour 100% de sa masse :
(1)- de 15% à 45% en masse de glycérol ;
(2)- de 30% en masse à 70% en masse de la (C_{R}) représentée par la formule (II) telle que définie précédemment ;
(3)- de 5% à 20% en masse de rhamnose.

Selon un autre aspect particulier, l'invention a pour objet la composition C₁ telle que définie précédemment, comprenant pour 100% de sa masse :
(1)- de 5% à 15% en masse de glycérol ;
(2)- de 65% en masse à 85% en masse de la (C_{R}) représentée par la formule (II) telle que définie précédemment ;
(3)- de 3% à 10% en masse de rhamnose.

La composition (C₁) telle que définie précédemment comprend aussi des produits secondaires non identifiés issus de la réaction d'acétalisation du sucre. Cependant ils ne représentent pas plus de 20% en masse de ladite composition (C₁).

La composition (C₁) telle que définie précédemment peut aussi optionnellement comprendre de l'eau, principalement en une proportion inférieure ou égale à 5% de sa masse.

L'invention a aussi pour un procédé de préparation de la composition (C₁) telle que définie précédemment caractérisé en ce qu'il comprend les étapes successives suivantes :
Une **étape a)** de chauffage sous agitation douce du glycérol jusqu'à le porter à une température supérieure (T₁) d'au moins 5°C au-dessus de sa température de fusion ;
Une **étape b)** de dispersion et d'homogénisation sous agitation de rhamnose monohydraté dans le milieu précédemment fondu dans le rapport stœchiométrique souhaité ;
Une **étape c)** d'acétalisation en ajoutant sous agitation dans le milieu liquide issu de l'étape b) une quantité catalytique d'acide fort, en maintenant le mélange réactionnel tout sous vide partiel et en distillant l'eau formée ; et si nécessaire ou si désiré,
Une **étape d)** de neutralisation de la solution obtenue à l'issue de l'étape d), pour obtenir ladite composition (C₁).

Pour la mise en œuvre de l'étape c) telle que définie ci-dessus, l'acide fort est notamment choisi parmi les acides sulfurique, chlorhydrique, phosphorique, nitrique, hypophosphoreux, méthane-sulfonique, para- toluène sulfonique, trifluorométhane sulfonique et les résines échangeuses d'ions acides. Habituellement, la réaction d'acétalisation est effectuée à une température (T₂) de 70°C à 130°C, sous un vide de 300 à 20 10² Pa (300 à 20 mbar).

La composition (C₁) telle que définie précédemment, peut être incorporée dans tout type de composition cosmétiques ou pharmaceutiques destinées à être appliquées sur la peau ou les muqueuses.

C'est pourquoi l'invention a aussi pour objet une composition cosmétique ou dermocosmétique topique (C), caractérisée en ce qu'elle comprend une quantité efficace de la composition (C₁) telle que définie précédemment.

L'invention a aussi pour objet une composition pharmaceutique ou dermopharmaceutique topique (C'), caractérisée en ce qu'elle comprend une quantité efficace de la composition (C₁) telle que définie précédemment.

Par « quantité efficace », on désigne, dans les définitions des compositions (C) et (C') susmentionnées, une proportion massique généralement supérieure ou égale à 0,5% en masse et inférieure ou égale à 5%, de leur masse totale et plus particulièrement de 1% en masse à 3% en masse de leur masse totale.

L'adjectif "topique" qualifiant des compositions (C) et (C') susmentionnées, signifie que ces dernières sont mises en œuvre par application sur la peau, le cuir chevelu ou les muqueuses.

Lesdites compositions (C) et (C') susmentionnées se présentent notamment sous la forme d'une solution aqueuse ou huileuse, d'une émulsion ou d'une microémulsion du type eau dans huile (E/H) ou huile-dans-eau (H/E), d'une émulsion multiple de type eau-dans-huile-dans-eau (E/H/E) ou huile-dans-eau-dans-huile (H/E/H), d'un gel, d'un savon ou d'un syndet, d'un baume, d'une hydro-dispersion, d'une crème, d'une mousse, d'un aérosol ou encore sous forme anhydre comme une poudre. Elles peuvent être utilisées en tant que crème, lait, bain moussant, shampooing, après-shampooing ou lotion pour le soin ou pour la protection du visage, des mains et du corps, plus particulièrement pour leur effet hydratant à court terme de l'épiderme après une exposition prolongée aux basses températures, ou aux rayonnements solaires ; ou bien pour prévenir ou ralentir l'apparition des signes extérieurs du vieillissement de la peau humaine, comme par l'apparition des rides, des ridules, d'une altération du microrelief, du manque d'élasticité et/ou de tonus, du manque de densité et/ou de fermeté de la peau humaine ; ou en encore après le rasage du visage ou pour le lavage et/ou du traitement du cuir chevelu.

De façon générale lesdites compositions (C) et (C'), comportent également des excipients et/ou des principes actifs habituellement mis en œuvre dans le domaine des formulations topiques, comme les tensioactifs moussants et/ou détergents, les tensioactifs épaississants et/ou gélifiants, les agents épaississants et/ou gélifiants, les agents stabilisants, les composés filmogènes, les solvants et co-solvants, les agents hydrotropes, les agents plastifiants, les matières grasses, les huiles et les cires, les agents émulsionnants et co-émulsionnants, les agents opacificants, les agents nacrants, les agents surgraissants, les séquestrants, les agents chélatants, les agents antioxydants, les parfums, les huiles essentielles, les agents conservateurs, les agents conditionneurs, les agents blanchissants destinés à la décoloration des poils et de la peau, les principes actifs destinés à apporter une action traitante et/ou protectrice vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques, les agents répulsifs pour les insectes.

Comme exemples de tensioactifs moussants et/ou détergents, optionnellement présents dans lesdites compositions (C) et (C') telles que définies précédemment, on peut citer les tensioactifs moussants et/ou détergents anioniques, cationiques, amphotères ou non ioniques topiquement acceptables habituellement utilisés dans ce domaine d'activité.

Parmi les tensioactifs anioniques moussants et/ou détergents optionnellement présents dans lesdites compositions (C) et (C') telles que définies précédemment on peut citer les sels de métaux alcalins, les sels de métaux alcalino-terreux, les sels d'ammonium, les sels d'amines, les sels d'amino-alcools, d'alkyléthers sulfates, d'alkylsulfates, d'alkylamidoéthersulfates, d'alkylaryl polyéthersulfates, de monoglycérides sulfates, d'alpha-oléfinesulfonates, de paraffines sulfonates, d'alkylphosphates, d'alkylétherphosphates, d'alkylsulfonates, d'alkylamidesulfonates, d'alkylarylsulfonates, d'alkylcarboxylates, d'alkylsulfosuccinates, d'alkyléthersulfosuccinates, d'alkylamidesulfosuccinates, d'alkylsulfoacétates, d'alkylsarcosinates, d'acyliséthionates, de N-acyltaurates, d'acyllactylates, de dérivés N-acylés d'acides aminés, de dérivés N-acylés de peptides, de dérivés N-acylés de protéines, d'acides gras.

Parmi les tensioactifs amphotères moussants et/ou détergents optionnellement présents dans lesdites compositions (C) et (C') telles que définies précédemment, il y a les alkylbétaines, les alkylamidobétaines, les sultaines, les alkylamidoalkylsulfobétaines, les dérivés d'imidazolines, les phosphobétaïnes, les amphopolyacétates et les amphopropionates.

Parmi les tensioactifs cationiques moussants et/ou détergents optionnellement présents dans lesdites compositions (C) et (C') telles que définies précédemment, il y a particulièrement les dérivés d'ammoniums quaternaires.

Parmi les tensioactifs non ioniques moussants et/ou détergents optionnellement présents dans lesdites compositions (C) et (C') telles que définies précédemment, il y a plus particulièrement les alkyl-polyglycosides, les dérivés d'huile de ricin, les polysorbates, les amides de coprah, les N-alkylamines.

Parmi les tensioactifs non ioniques moussants et/ou détergents optionnellement présents dans lesdites compositions (C) et (C') telles que définies précédemment, il y a plus particulièrement la composition (C₃) ou un mélange de compositions (C₃), ladite composition (C₃) étant représentée par la formule (IV) :

R₂-O-(G₂)ₚ-H (IV)

dans laquelle R₂ représente un radical aliphatique linéaire ou ramifié, saturé ou insaturé, comportant de 8 à 16 atomes de carbone, G₂ représente le reste d'un sucre réducteur et p représente un nombre décimal supérieur ou égal à 1,05 et inférieur ou égal à 5, ladite composition (C₃) consistant essentiellement en un mélange de composés représentés par les formules (IV₁), (IV₂), (IV₃), (IV₄) et (IV₅) :

R₂-O-(G₂)₁-H (IV₁),

R₂-O-(G₂)₂-H (IV₂),

R₂-O-(G₂)₃-H (IV₃),

R₂-O-(G₂)₄-H (IV₄),

R₂-O-(G₂)₅-H (IV₅),

dans les proportions molaires respectives a₁, a₂, a₃, a₄ et a₅, telles que la somme a₁+a₂+a₃+a₄+a₅ est égale à 1 et que la somme a₁+2a₂+3a₃+4a₄+5a₅ est égale à p.

Comme exemples de tensioactifs épaississants et/ou gélifiants optionnellement présents dans lesdites compositions (C) et (C') telles que définies précédemment, on peut citer les esters gras d'alkyl-polyglycosides optionnellement alkoxylés, et tout particulièrement les esters de méthylpolyglucoside éthoxylés tels que le PEG 120 méthyl glucose trioléate ou le PEG 120 méthyl glucose dioléate commercialisés respectivement sous les nom GLUCAMATE™LT et GLUMATE™DOE120 ; les esters gras alkoxylés tels que le PEG 150 pentaérythrytyl tétrastéarate commercialisé sous l'nom CROTHIX™DS53, le PEG 55 propylene glycol oléate commercialisé sous l'appellation ANTIL™141 ; les carbamates de polyalkylène glycols à chaînes grasses tels que le PPG 14 laureth isophoryl dicarbamate commercialisé sous l'nom ELFACOS™T211, le PPG 14 palmeth 60 hexyl dicarbamate commercialisé sous l'nom ELFACOS™GT2125.

Comme exemples de tensioactifs émulsionnants optionnellement présents dans lesdites compositions (C) et (C'), telles que définies précédemment, on peut citer des tensioactifs non ioniques, des tensioactifs anioniques, des tensioactifs cationiques.

Comme exemples de tensioactifs non ioniques émulsionnants optionnellement présents dans lesdites compositions (C) et (C'), telles que définies précédemment on peut citer les esters d'acides gras et de sorbitol, par exemple les MONTANE™80, MONTANE™85 et MONTANE™60 ; les alkyl polyglycosides et les compositions d'alkyl polyglycosides et d'alcools gras linéaires ou ramifiés, saturés ou insaturés, la chaîne alkyles desdits alkyl polyglycosides étant constituée par des groupements alkyles linéaires ou ramifiés, saturés ou insaturés, comportant de 14 à 22 atomes de carbone, par exemple les MONTANOV™, EASYNOV™ et FLUIDANOV™ ; les esters d'acide gras et de polyglycérol, par exemple ISOLAN™ GI34 et PLUROL™ DIISOSTEARIQUE ; l'huile de ricin éthoxylée et l'huile de ricin hydrogénée éthoxylée, le SIMULSOL™989 ; les compositions comprenant du stéarate de glycérol et de d'acide stéarique éthoxylé entre 5 moles et 150 moles d'oxyde d'éthylène, par exemple la composition comprenant de l'acide stéarique éthoxylé à 135 moles d'oxyde d'éthylène et du stéarate de glycérol commercialisée sous le nom SIMULSOL™ 165 ; les polyhydroxystéarates de polyglycol ou de polyglycérol par exemple l'HYPERMER™B246 ou l'ARLACEL™P135, le DEHYMULS™PGPH, le DECAGLYN™5HS ; les copolymères polyéthylèneglycol-alkylglycol comme le PEG-45 dodécylglycol copolymère tel que l'ELFACOS™ST 9 ; les esters de sorbitan éthoxylés, par exemple les MONTANOX™ ; les esters de mannitan ; les esters de mannitan éthoxylés ; les esters de sucrose ; les esters de méthylglucoside.

Comme exemples de tensioactifs anioniques émulsionnants optionnellement présents dans lesdites compositions (C) et (C') telles que définies précédemment, on peut citer le décylphosphate, le cétylphosphate commercialisé sous le nom AMPHISOL™, le glycéryl stéarate citrate ; le cétéarylsulfate ; la composition arachidyl/béhényl phosphates et arachidyl/béhényl alcools commercialisée sous le nom SENSANOV™WR; les savons comme par exemple le stéarate de sodium ou le stéarate de triéthanolammonium, les dérivés N-acylés d'acides aminés salifiés comme par exemple le stéaroyl glutamate.

Comme exemples de tensioactifs cationiques émulsionnants optionnellement présents dans lesdites compositions (C) et (C') telles que définies précédemment, on peut citer les aminoxydes, le QUATERNIUM™82 et les tensioactifs décrits dans la demande internationale publiée sous le numéro WO 96/00719 et principalement ceux dont la chaîne grasse comprend au moins 16 atomes de carbone.

Comme exemples d'agents opacifiants et/ou nacrants optionnellement présents dans lesdites compositions (C) et (C') telles que définies précédemment, on peut citer le palmitate de sodium, le stéarate de sodium, l'hydroxystéarate de sodium, le palmitate de magnésium, le stéarate de magnésium, l'hydroxystéarate de magnésium, le monostéarate d'éthylène glycol, le distéarate d'éthylène glycol, le monostéarate de polyéthylène glycol, le distéarate de polyéthylène glycol, les alcools gras comportant de 12 à 22 atomes de carbone.

Comme exemples d'agents de texture optionnellement présents dans lesdites compositions (C) et (C') telles que définies précédemment, on peut citer des dérivés N-acylés d'acides aminés, par exemple la lauroyl lysine commercialisée sous le nom AMlNOHOPE™LL, l'octenyl starch succinate commercialisé sous le nom DRYFLO™, le myristyl polyglucoside commercialisé sous le nom MONTANOV™14, les fibres de cellulose, les fibres de coton, les fibres de chitosane, le talc, la séricite, le mica.

Comme exemples de solvants et de cosolvants optionnellement présents dans lesdites compositions (C) et (C') telles que définies précédemment, on peut citer l'eau, l'éthylène glycol, le propylène glycol, le butylène glycol, l'hexylène glycol, le diéthylène glycol, les alcools hydrosolubles tels que l'éthanol, l'isopropanol ou le butanol, les mélanges d'eau et desdits solvants.

Comme exemples d'huiles optionnellement présentes dans lesdites compositions (C) et (C') telles que définies précédemment, on peut citer les huiles minérales telles que l'huile de paraffine, l'huile de vaseline, les isoparaffines ou les huiles blanches minérales ; les huiles d'origine animale, telles que le squalène ou le squalane ; les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes ; les huiles végétales éthoxylées ; les huiles synthétiques comme les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les poly(alpha-oléfine), les polyoléfines comme le poly(isobutane), les isoalcanes de synthèse comme l'isohexadécane, l'isododécane, les huiles perfluorées ; les huiles de silicone comme les diméthylpolysiloxanes, les méthylphényl - polysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles. Par « huiles », on entend dans la présente demande les composés et/ou les mélanges de composés insolubles dans l'eau, se présentant sous un aspect liquide à une température de 25°C.

Comme exemples de cires optionnellement présentes dans lesdites compositions (C) et (C') telles que définies précédemment, on peut citer la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline ; l'ozokérite ; la cire de polyéthylène ; les cires de silicone ; les cires végétales ; les alcools gras et les acides gras solides à température ambiante ; les glycérides solides à température ambiante. Par « cires », on entend dans la présente demande les composés et/ou les mélanges de composés insolubles dans l'eau, se présentant sous un aspect solide à une température supérieure ou égale à 45°C.

Comme exemples de matières grasses optionnellement présentes dans lesdites compositions (C) et (C') telles que définies précédemment, on peut citer les alcools gras saturés ou insaturés, linéaires ou ramifiés, comportant de huit à trente-six atomes de carbone, ou les acides gras saturés ou insaturés, linéaires ou ramifiés, comportant de huit à trente-six 36 atomes de carbone.

Comme exemples d'agents épaississants et/ou gélifiants optionnellement présents dans lesdites compositions (C) et (C') telles que définies précédemment, il y a les polymères de type polyélectrolytes, linéaires ou branchés ou réticulés, comme l'homopolymère de l'acide acrylique partiellement ou totalement salifié, l'homopolymère de l'acide méthacrylique partiellement ou totalement salifié, l'homopolymère de l'acide-2-méthyl-[(1-oxo-2-propényl)amino]-1-propanesulfonique (AMPS) partiellement ou totalement salifié, les copolymères de l'acide acrylique et de l'AMPS, les copolymères de l'acrylamide et de l'AMPS, les copolymères de la vinylpyrolidone et de l'AMPS, les copolymères de l'AMPS et de l'acrylate de (2-hydroxyéthyle), les copolymères de l'AMPS et du méthacrylate de (2-hydroxyéthyle), les copolymères de l'AMPS et de l'hydroxyéthylacrylamide, les copolymères de l'AMPS et du N,N-diméthyl acrylamide, les copolymères de l'AMPS et du tris(hydroxy-methyl)acrylamido methane (THAM), les copolymères de l'acide acrylique ou méthacrylique et de l'acrylate de (2-hydroxy éthyle), les copolymères de l'acide acrylique ou méthacrylique et du méthacrylate de (2-hydroxy éthyle), les copolymères de l'acide acrylique ou méthacrylique et de l'hydroxyéthylacrylamide, les copolymères de l'acide acrylique ou méthacrylique et du THAM, les copolymères de l'acide acrylique ou méthacrylique et du N,N-diméthyl acrylamide, les terpolymères de l'acide acrylique ou méthacrylique, de l'AMPS et de l'acrylate de (2-hydroxy éthyle), les terpolymères de l'acide acrylique ou méthacrylique, de l'AMPS et du méthacrylate de (2-hydroxy éthyle), les terpolymères de l'acide acrylique ou méthacrylique, de l'AMPS et du THAM, les terpolymères de l'acide acrylique ou méthacrylique, de l'AMPS et du N,N-diméthyl acrylamide, les terpolymères de l'acide acrylique ou méthacrylique, de l'AMPS et de l'acrylamide, les copolymères de l'acide acrylique ou de l'acide méthacrylique et d'acrylates d'alkyle dont la chaîne carbonée comprend entre quatre et trente atomes de carbone et plus particulièrement entre dix et trente atomes de carbone, les copolymères de l'AMPS et d'acrylates d'alkyle dont la chaîne carbonée comprend entre quatre et trente atomes de carbone et plus particulièrement entre dix et trente atomes de carbone, les terpolymères linéaires, branchés ou réticulés d'au moins un monomère possédant une fonction acide fort, libre, partiellement salifiée ou totalement salifiée, avec au moins un monomère neutre, et au moins un monomère de formule (V) :

CH₂=C(R₇)-C(=O)-[CH₂-CH₂-O]_{z}-R₈ (V)

dans laquelle R₇ représente un atome d'hydrogène ou un radical méthyle, R₈ représente un radical alkyle linéaire ou ramifié comportant de huit à trente atomes de carbone et z représente un nombre supérieur ou égal à un et inférieur ou égal à cinquante.

Les polymères de type polyélectrolytes, linéaires ou branchés ou réticulés optionnellement présents à lesdites compositions (C) et (C'), telles que définies précédemment peuvent se présenter sous la forme d'une solution, d'une suspension aqueuse, d'une émulsion eau-dans-huile, d'une émulsion huile-dans-eau, d'une poudre. Les polymères de type polyélectrolytes, linéaires ou branchés ou réticulés optionnellement présents à lesdites compositions (C) et (C'), telles que définies précédemment peuvent être sélectionnés parmi les produits commercialisés sous les appellations SIMULGEL™ EG, SIMULGEL™EPG, SEPIGEL™ 305, SIMULGEL™ 600, SIMULGEL™NS, SIMULGEL™ INS 100, SIMULGEL™ FL, SIMULGEL™ A, SIMULGEL™ SMS 88, SEPINOV™EMT 10, SEPIPLUS™400, SEPIPLUS™265, SEPIPLUS™S, SEPIMAX™Zen, ARISTOFLEX™AVC, ARISTOFLEX™AVS, NOVEMER™EC-1, NOVEMER™EC 2, ARISTOFLEX™HMB, COSMEDIA™SP, FLOCARE™ET 25, FLOCARE™ET 75, FLOCARE™ET 26, FLOCARE™ET 30, FLOCARE™ET 58, FLOCARE™PSD 30, VISCOLAM™AT 64, VISCOLAM™AT 100.

Comme exemples d'agents épaississants et/ou gélifiants optionnellement présents dans lesdites compositions (C) et (C') telles que définies précédemment, il y a les polysaccharides constitués uniquement d'oses, comme les glucanes ou homopolymères du glucose, les glucomannoglucanes, les xyloglycanes, les galactomannanes dont le degré de substitution (DS) des unités de D-galactose sur la chaîne principale de D-mannose est compris entre 0 et 1, et plus particulièrement entre 1 et 0,25, comme les galactomannanes provenant de la gomme de cassia (DS = 1/5), de la gomme de caroube (DS = 1/4), de la gomme de tara (DS = 1/3), de la gomme de guar (DS = 1/2), de la gomme de fenugrec (DS = 1 ).

Comme exemples d'agents épaississants et/ou gélifiants optionnellement présents dans lesdites compositions (C) et (C') telles que définies précédemment, on peut citer les polysaccharides constitués de dérivés d'oses, comme les galactanes sulfatés et plus particulièrement les carraghénanes et l'agar, les uronanes et plus particulièrement les algines, les alginates et les pectines, les hétéropolymères d'oses et d'acides uroniques et plus particulièrement la gomme xanthane, la gomme gellane, les exsudats de gomme de arabique et de gomme de karaya, les glucosaminoglycanes.

Comme exemples d'agents épaississants et/ou gélifiants optionnellement présents dans lesdites compositions (C) et (C') telles que définies précédemment, il y a la cellulose, les dérivés de cellulose comme la méthyl-cellulose, l'éthyl-cellulose, l'hydroxypropyl cellulose, les silicates, l'amidon, les dérivés hydrophiles de l'amidon, les polyuréthanes.

Comme exemples d'agents stabilisants optionnellement présents dans lesdites compositions (C) et (C') telles que définies précédemment, il y a les cires microcristallines, et plus particulièrement l'ozokérite, les sels minéraux tels que le chlorure de sodium ou le chlorure de magnésium, les polymères siliconés tels que les copolymères polysiloxane polyalkyl polyéther.

Comme exemples d'eaux thermales ou minérales optionnellement présents dans lesdites compositions (C) et (C') telles que définies précédemment, il y a les eaux thermales ou minérales ayant une minéralisation d'au moins 300 mg/l, en particulier l'eau d'Avene, l'eau de Vittel, les eaux du bassin de Vichy, l'eau d'Uriage, l'eau de la Roche Posay, l'eau de la Bourboule, l'eau d'Enghien-les-bains, l'eau de Saint-Gervais-les bains, l'eau de Néris-les-bains, l'eau d'Allevard-les-bains, l'eau de Digne, l'eau des Maizieres, l'eau de Neyrac-les-bains, l'eau de Lons le Saunier, l'eau de Rochefort, l'eau de Saint Christau, l'eau des Fumades et l'eau de Tercis-les-bains.

Comme exemples d'agents hydrotropes optionnellement présents dans lesdites compositions (C) et (C') telles que définies précédemment, il y a les xylènes sulfonates, les cumènes sulfonates, l'hexyl polyglucoside, le (2-éthyl hexyl) polyglucoside, le n-heptyl polyglucoside.

Comme exemples d'agents déodorants optionnellement présents dans lesdites compositions (C) et (C') telles que définies précédemment, il y a les silicates alcalins, les sels de zinc comme le sulfate de zinc, le gluconate de zinc, le chlorure de zinc, le lactate de zinc ; les sels d'ammonium quaternaires comme les sels de cétyltriméthylammonium, les sels de cétylpyridinium ; les dérivés du glycérol comme le caprate de glycérol , le caprylate de glycérol, le caprate de polyglycérol ; le 1,2 décanediol ; le 1,3 propanediol ; l'acide salicylique ; le bicarbonate de sodium ; les cyclodextrines ; les zéolithes métalliques ; le TRICLOSAN™ ; le bromohydrate d'aluminium, les chlorhydrates d'aluminium, le chlorure d'aluminium, le sulfate d'aluminium, les chlorhydrates d'aluminium et de zirconium, le trichlorhydrate d'aluminium et de zirconium, le tétrachlorhydrate d'aluminium et de zirconium, le pentachlorhydrate d'aluminium et de zirconium, l'octochlorhydrate d'aluminium et de zirconium, le sulfate d'aluminium, le lactate de sodium et d'aluminium, les complexes de chlorhydrate d'aluminium et de glycol, comme le complexe de chlorhydrate d'aluminium et de propylène glycol, le complexe de dichlorhydrate d'aluminium et de propylène glycol, le complexe de sesquichlorhydrate d'aluminium et de propylène glycol, le complexe de chlorhydrate d'aluminium et de polyéthylène glycol, le complexe de dichlorhydrate d'aluminium et de polyéthylène glycol, le complexe de sesquichlorhydrate d'aluminium et de polyéthylène glycol.

Comme exemples de filtres solaires optionnellement présents dans lesdites compositions (C) et (C') telles que définies précédemment, il y a tous ceux figurant dans la directive cosmétique 76/768/CEE modifiée annexe VII.

Parmi les filtres organiques solaires optionnellement présents dans lesdites compositions (C) et (C') telles que définies précédemment, il y a la famille des dérivés de l'acide benzoïque comme les acides para-amino benzoïques (PABA), notamment les esters de monoglycérol de PABA, les esters éthyliques de N,N-propoxy PABA, les esters éthyliques de N,N-diéthoxy PABA, les esters éthyliques de N,N-diméthyl PABA, les esters méthyliques de N,N-diméthyl PABA, les esters butyliques de N,N-diméthyl PABA; la famille des dérivés de l'acide anthranilique comme l'homomenthyl-N-acétyl anthranilate ; la famille des dérivés de l'acide salicylique comme le salicylate d'amyle, le salicylate d'homomenthyle, le salicylate d'éthyl hexyle, le salicylate de phényle, le salicylate de benzyle, le salicylate de p-isopropyl phényle ; la famille des dérivés de l'acide cinnamique comme le cinnamate d'éthylhexyle, le cinnamate d'éthyl-4-isopropyle, le cinnamate de méthyl-2,5-diisopropyle, le cinnamate de p-méthoxypropyle, le cinnamate de p-méthoxyisopropyle, le cinnamate de p-méthoxyisoamyle, le cinnamate de p-méthoxyoctyle (le cinnamate de p-méthoxy 2-éthylhexyle), le cinnamate de p-méthoxy 2-éthoxyéthyle, le cinnamate de p-méthoxycyclohexyle, le cinnamate d'éthyl-α-cyano-β-phényle, le cinnamate de 2-éthylhexyl-α-cyano-β-phényle, le cinnamate de diparaméthoxy mono-2-éthylhexanoyl de glycéryle ; la famille des dérivés de la benzophénone comme la 2,4-dihydroxy benzophénone, la 2,2'-dihydroxy 4-méthoxy benzophénone, la 2,2',4,4'-tétrahydroxy benzophénone, la 2-hydroxy 4-méthoxy benzophénone, la 2-hydroxy 4-méthoxy 4'-méthyl benzophénone, la 2-hydroxy 4-méthoxy benzophénone-5-sulfonate, la 4-phényl benzophénone, le 4'-phényl benzophénone-2-carboxylate de (2-éthyl hexyle), la 2-hydroxy 4-(octyloxy) benzophénone, la 4-hydroxy benzophénone-3-carboxylique ; le 3-(4'-méthylbenzylidène)-d,l-camphre, le 3-(benzylidène)-d,l-camphre, le benzalkonium méthosulfate camphre ; l'acide urocanique, l'urocanate d'éthyle ; la famille des dérivés de l'acide sulfonique comme l'acide 2-phényl benzimidazole-5-sulfonique et ses sels ; la famille des dérivés de la triazine comme l'hydroxyphényl triazine, l'(éthyl hexyloxy) (hydroxy) phényl 4-méthoxyphényl triazine, le 2,4,6-trianillino-(p-carbo-2'-éthyl hexyl-1'-oxy)-1,3,5-triazine, le 4,4-((6-(((1,1-diméthyléthyl) amino) carbonyl) phenyl) amino)-1,3,5-triazine-2,4-diyl diimino) bis-(2-éthylhexyl) ester de l'acide benzoïque, le 2-phényl 5-méthyl benzoxazole, le 2,2'-hydroxy-5-méthyl phényl benzotriazole, le 2-(2'-hydroxy-5'-t-octylphényl) benzotriazole, le 2-(2'-hydroxy-5'-méthyphényl)benzotriazole; la dibenzazine; le dianisoylméthane, le 4-méthoxy-4"-t-butylbenzoylméthane ; la 5-(3,3-diméthyl-2-norbornylidène)-3-pentan-2-one ; la famille des dérivés du diphénylacrylate comme le 2-éthylhexyl-2-cyano-3,3-diphényl-2-propènoate, l'éthyl-2-cyano-3,3-diphényl-2-propènoate ; la famille des polysiloxanes comme le malonate de benzylidène siloxane.

Parmi les filtres inorganiques solaires, également appelés "écrans minéraux", optionnellement présents dans lesdites compositions (C) et (C') telles que définies précédemment, il y a les oxydes de titane, les oxydes de zinc, l'oxyde de cérium, l'oxyde de zirconium, les oxydes de fer jaune, rouge ou noir, les oxydes de chrome. Ces écrans minéraux peuvent être micronisés ou non, avoir subi ou non des traitements de surface et être optionnellement présentés sous formes de pré-dispersions aqueuses ou huileuses.

Comme exemples de principes actifs optionnellement présents dans lesdites compositions (C) et (C') telles que définies précédemment, il y a les vitamines et leurs dérivés, notamment leurs esters, tels que le rétinol (vitamine A) et ses esters (palmitate de rétinyle par exemple), l'acide ascorbique (vitamine C) et ses esters, les dérives de sucre de l'acide ascorbique (comme l'ascorbyl glucoside), le tocophérol (vitamine E) et ses esters (comme l'acétate de tocophérol), les vitamines B3 ou B10 (niacinamide et ses dérivés) ; les composés montrant une action éclaircissante ou dépigmentante de la peau comme le ω-undecelynoyl phénylalanine commercialisé sous l'appellation SEPIWHITE™MSH, le SEPICALM™VG, le mono ester et/ou le diester de glycérol du ω-undecelynoyl phénylalanine, les ω-undecelynoyl dipeptides, l'arbutine, l'acide kojique, l'hydroquinone ; les composés montrant une action apaisante notamment le SEPICALM™ S, l'allantoïne et le bisabolol ; les agents anti-inflammatoires ; les composés montrant une action hydratante comme l'urée, les hydroxyurées, le glycérolglucoside, le diglycérolglucoside, les polyglycérylglucosides ; les extraits végétaux riches en polyphénols comme les extraits de raisin, les extraits de pin, les extraits de vin, les extraits d'olives ; les composés montrant une action amincissante ou lipolytique comme la caféine ou ses dérivés, l'ADIPOSLIM™, l'ADIPOLESS™, la fucoxanthine ; les protéines N-acylées ; les peptides N-acylés comme le MATRIXIL™ ; les acides aminés N―acylés ; les hydrolysâts partiels de protéines N-acylés ; les acides aminés ; les peptides ; les hydrolysâts totaux de protéines ; les extraits de soja, par exemple la Raffermine™ ; les extraits de blé par exemple la TENSlNE™ ou la GLIADINE™ ; les extraits végétaux, tels que les extraits végétaux riches en tanins, les extraits végétaux riches en isoflavones ou les extraits végétaux riches en terpènes ; les extraits d'algues d'eau douce ou marines ; les extraits de plantes marines ; les extraits marins en général comme les coraux ; les cires essentielles ; les extraits bactériens ; les céramides ; les phospholipides ; les composés montrant une action antimicrobienne ou une action purifiante, comme le LIPACIDE™ C8G, le LIPACIDE™ UG, le SEPICONTROL™ A5 ; l'OCTOPIROX™ ou le SENSIVA™ SC50 ; les composés montrant une propriété énergisante ou stimulante comme le PHYSIOGENYL™, le panthénol et ses dérivés comme le SEPICAP™ MP ; les actifs anti-âge comme le SEPILIFT™ DPHP, le LIPACIDE™ PVB, le SEPIVINOL™, le SEPIVITAL™, le MANOLIVA™, le PHYTO-AGE™, le TIMECODE™ ; le SURVICODE™ ; les actifs anti-photo vieillissement ; les actifs protecteurs de l'intégrité de la jonction dermo-épidermique ; les actifs augmentant la synthèse des composants de la matrice extracellulaire comme le collagène, les élastines, les glycosaminoglycanes ; les actifs agissant favorablement sur la communication cellulaire chimique comme les cytokines ou physiques comme les intégrines ; les actifs créant une sensation de « chauffe » sur la peau comme les activateurs de la microcirculation cutanée (comme les dérivés de l'acide nicotinique) ou des produits créant une sensation de « fraîcheur » sur la peau (comme le menthol et des dérivés) ; les actifs améliorant la microcirculation cutanée, par exemple les veinotoniques ; les actifs drainants ; les actifs à visée décongestionnante comme les extraits de ginko biloba, de lierre, de marron d'inde, de bambou, de ruscus, de petit houx, de *centalla asiatica,* de fucus, de romarin, de saule ; les agents de bronzage ou de brunissement de la peau, comme par exemple la dihydroxyacétone (DHA), l'érythrulose, l'aldéhyde mésotartrique, le glutaraldéhyde, le glycéraldéhyde, l'alloxane, la ninhydrine, les extraits végétaux comme par exemple les extraits de bois rouges du genre Pterocarpus et du genre Baphia comme le Pteropcarpus santalinus, le Pterocarpus osun, le Pterocarpus soyauxii, le Pterocarpus erinaceus, le Pterocarpus indicus ou le Baphia nitida comme ceux décrits dans la demande de brevet européen EP 0 971 683 ; ; les agents connus pour leur action de facilitation et/ou d'accélération du bronzage et/ou du brunissement de la peau humaine, et/ou pour leur action de coloration de la peau humaine, comme par exemple les caroténoïdes ( et plus particulièrement le beta carotène et le gamma carotène), le produit commercialisé sous le nom de marque « Carrot oil » (Nom INCI : Daucus Carota, helianthus annuus Sunflower oil) par la société Provital, qui contient des caroténoïdes, de la vitamine E et de la vitamine K ; la tyrosine et/ou ses dérivés, connus pour leur effet sur l'accélération du bronzage de la peau humaine en association avec une exposition aux rayonnements ultra-violets, comme par exemple le produit commercialisé sous le nom de marque « SunTan Accelerator™ » par la société Provital qui contient de la tyrosine et des riboflavines (vitamine B), le complexe de tyrosine et de tyrosinase commercialisé sous le nom de marque « Zymo Tan Complex » par la société Zymo Line, le produit commercialisé sous le nom de marque MelanoBronze™ (nom INCI : Acetyl Tyrosine, Monk's pepper extract (Vitex Agnus-castus)) par la société Mibelle qui contient de l'acétyl tyrosine, produit commercialisé sous le nom de marque Unipertan VEG-24/242/2002 (nom INCI : butylene glycol and Acetyl Tyrosine and hydrolyzed vegetable protein and Adenosine triphosphate) par la société UNIPEX, le produit commercialisé sous le nom de marque « Try-Excell™ » (nom INCI : Oleoyl Tyrosine and Luffa Cylindrica (Seed) Oil and Oleic acid) par la société Sederma qui contient des extraits de pépins de courge (ou huile de Loofah), le produit commercialisé sous le nom de marque «Actibronze™ » (nom INCI : hydrolyzed wheat protein and acetyl tyrosine and copper gluconate) par la société Alban Muller, le produit commercialisé sous le nom de marque Tyrostan™ (nom INCI : potassium caproyl tyrosine) par la société Synerga, le produit commercialisé sous le nom de marque Tyrosinol (nom INCI : Sorbitan Isostearate, glyceryl oleate, caproyl Tyrosine) par la société Synerga, le produit commercialisé sous le nom de marque InstaBronze™ (nom INCI : Dihydroxyacetone and acetyl tyrosine and copper gluconate) commercialisé par la société Alban Muller, le produit commercialisé sous le nom de marque Tyrosilane (nom INCI : méthylsilanol and acétyl tyrosine) par la société Exymol ; les peptides connus pour leur effet d'activation de la mélanogénèse comme par exemple le produit commercialisé sous le nom de marque Bronzing SF Peptide powder (nom INCI : Dextran and Octapeptide-5) par la société Infinitec Activos, le produit commercialisé sous le nom de marque Melitane (nom INCI : Glycerin and Aqua and Dextran and Acetyl hexapeptide-1 ) comprenant l'acétyl hexapeptide-1 connu pour son action agoniste de l'alpha-MSH, le produit commercialisé sous le nom de marque Melatimes Solutions™ (nom INCI : Butylene glycol , Palmitoyl Tripeptide-40) par la société LIPOTEC, les sucres et les dérivés de sucres comme par exemple le produit commercialisé sous le nom de marque Tanositol™ (nom INCI : inositol) par la société Provital, le produit commercialisé sous le nom de marque Thalitan™ (ou Phycosaccharide™ AG) par la société CODIF international (nom INCI : Aqua and hydrolyzed algin (Laminaria Digitata) and magnesium sulfate and manganese sulfate) contenant un oligosaccharide d'origine marine (acide guluronique et acide mannuronique chélatés avec les ions magnésium et manganèse), le produit commercialisé sous le nom de marque Melactiva™ (nom INCI : Maltodextrin, Mucuna Pruriens Seed extract) par la société Alban Muller, les composés riches en flavonoïdes comme par exemple le produit commercialisé sous le nom de marque « Biotanning » (nom INCI : Hydrolyzed citrus Aurantium dulcis fruit extract) par la société Silab et connu pour être riche en flavonoides de citron (de type hespéridines).

Comme exemples d'agents antioxydants optionnellement présents dans lesdites compositions (C) et (C') telles que définies précédemment, il y a l'EDTA et ses sels, l'acide citrique, l'acide tartrique, l'acide oxalique, le BHA (butylhydroxyanisol), le BHT (butylhydroxytoluène), les dérivés de tocophérol tels que l'acétate de tocophérol, des mélanges de composés antioxydants tels que la DISSOLVINE™ GL 47S commercialisé par la société Akzo Nobel sous le nom INCI : Tetrasodium Glutamate Diacetate.

Les exemples suivants illustrent l'invention sans toutefois la limiter.

### A) - Préparation de compositions C₁ selon l'invention.

Le mode opératoire général de la réaction de glycosylation mis en œuvre pour préparer les compositions selon l'invention est le suivant :
- Chargement du glycérol dans un réacteur équipé d'une agitation mécanique et d'un montage de distillation sous vide,
- Chargement du rhamnose monohydraté sous forme de poudre dans les proportions stoechiométriques indiquées dans les tableaux 1 et 2 suivants :
- Mise sous agitation et chauffage jusqu'à la fonte totale du rhamnose,
- Mise sous vide partiel pour éliminer l'eau contenue dans le rhamnose,
- Ajout du système catalytique, 1,0% massique d'H₃PO₂ (acide hypophosphorique) à 50% (par rapport à la masse de rhamnose),
- Chauffage jusqu'à réaction en quasi-totalité du rhamnose.
- Si nécessaire, neutralisation du milieu avec une solution comprenant un mélange soude et de borohydrure de sodium, en fonction de la viscosité du milieu réactionnel.

| Conditions opératoires | | |
|---|---|---|
| Proportion initiale en glycérol | 1 eq. molaire | 1 eq. molaire |
| Proportion initiale en rhamnose | 1,25 eq. molaire | 0,66 eq molaire |
| Catalyseur | H₃PO₂ | H₃PO₂ |
| Température d'acylation | 110-130°C | 110°C |

| Caractéristique de la composition obtenue (% en masse) | | |
|---|---|---|
| | Composition (C_{1A}) | Composition (C_{1B}) |
| Glycéryl polyrhamnoside | 64,5% | 33,1% |
| Degré de polymérisation moyen (*) | 1,51 | 1,17 |
| Glycérol | 20,2% | 41,3% |
| Rhamnose résiduel | 6,6% | 16,7% |
| Produits secondaires | 8,6% | 5,9% |
| Eau | 0,1% | 3% |

| Conditions opératoires | | |
|---|---|---|
| Proportion initiale en glycérol | 1 eq. molaire | 1 eq. Molaire |
| Proportion initiale de rhamnose | 2,00 eq. molaire | 3,33 eq. Molaire |
| Catalyseur | H₃PO₂ | H₃PO₂ |
| Température d'acylation | 135°C | 135°C |

| Caractéristique de la composition obtenue (% en masse) | | |
|---|---|---|
| | Composition (C_{1C}) | Composition (C_{1D}) |
| Glycéryl polyrhamnoside | 69,6% | 80,5% |
| Degré de polymérisation moyen^{(*)} | 1,68 | 2,01 |
| Glycérol | 11,9% | 5,9% |
| Rhamnose résiduel | 6,4% | 4,1% |
| Produits secondaires | 12% | 9,5% |
| Eau | 0,1% | 0% |

| | | |
|---|---|---|
| (*) : le degré de polymérisation moyen des polyols rhamnosides est obtenu à partir d'une chromatographie en phase gazeuse puis en : i) déterminant des pourcentages massiques de chaque oligomère, en ii) en normalisant à 100% des proportions massiques obtenues, en iii) en convertissant en pourcentages molaire les pourcentages massiques normalisés obtenus et en iv) calculant le degré moyen de polymérisation sur la base de chaque pourcentage molaire obtenu, pondéré par le nombre de motif rhamnose dans l'oligomère en question. | | |

### B) Formulations

Dans les formules suivantes, les pourcentages sont exprimés en poids de la formulation.

### B.1 Fluide démaquillant visage

**Formule**

| | |
|---|---|
| Composition (C_{1A}) | 10,00% |
| Méthyl paraben | 0,15% |
| Phenoxyethanol | 0,80% |
| SEPICALM™ S | 1,00% |
| Parfum/Fragrance | 0,10% |
| Eau | qs. 100,00% |

Mode opératoire : Mélanger les différents ingrédients dans l'eau sous agitation magnétique dans l'ordre indiqué, et ajuster le pH aux alentours de 7.

### B.2 Shampoing cheveux et corps pour enfants

**Formule**

| | | |
|---|---|---|
| A | Composition (C_{1B}) | 15,00% |
| | PROTEOL™APL | 5,00% |
| | SEPICIDE™HB | 0,50% |
| | Parfum/Fragrance | 0,10% |
| B | Eau | 20,00% |
| | CAPIGEL™98 | 3,50% |
| C | Eau | Q.S. 100,00% |
| | SEPlClDE™Cl | 0,30% |
| | Colorant | Q.S |
| | Soude | Q.S. pH = 7,2 |

Mode opératoire : Mélanger la composition (E₄) avec le PROTEOL™APL, et le SEPICIDE™HB (Phase A). Diluer le CAPIGEL™98 dans une partie de l'eau et l'ajouter à la phase A précédemment obtenue (Phase B). Ajouter le reste d'eau à la phase B, puis le SEPICIDE™CI et le colorant. Ajuster le pH du mélange à 7,2 environ avec de la soude.

### B.3 Lingettes démaquillantes pour les veux

**Formule**

| | | |
|---|---|---|
| A | Composition ((C_{1C}) | 3,00% |
| B | SEPICIDE™HB2 | 0,50% |
| C | SEPICALM™ VG | 0,50% |
| | Parfum/Fragrance | 0,05% |
| D | Eau | Q.S. 100,00% |

Mode opératoire : Mélanger les ingrédients de la phase B ainsi que ceux de la phase C dans la phase A jusqu'à obtenir la limpidité de la solution. Ajouter la phase D.

### B.4 Gel moussant doux

**Formule**

| | | |
|---|---|---|
| A | Composition (C_{1D})) | 8,50% |
| | PROTEOL™ APL | 3,00% |
| | EUXYL ™ PE9010 | 1,00% Parfum/Fragrance 0,10% |
| B | Eau | Q.S. 100,00% |
| | Acide lactique | Q.S. pH = 6,0 |

Mode opératoire : Solubiliser le parfum et le conservateur EUXYL™ PE 9010 dans le mélange composé de la composition E₄ et du PROTEOL™ APL (phase A). Ajouter l'eau et régler le pH à environ 6,0 avec de l'acide lactique.

### B.5 Shampoing à usage fréquent

**Formule**

| | | |
|---|---|---|
| A | Composition (C_{1C}) | 12,80% |
| | PROTEOL™ OAT | 5,00% |
| | EUXYL ™ PE 9010 | 1,00% |
| | Parfum/Fragrance | 0,30% |
| | Eau Q.S. | 100,00% |
| B | MONTALINE™C40 | 8,50% |
| | Acide lactique | Q.S. pH = 6,0 |

Mode opératoire : mélanger tous les ingrédients de la phase A et, après homogénéisation, ajouter la MONTALINE™C40 et ajuster le pH à environ 6,0 à l'aide de l'acide lactique.

### B.6 Shampoing ultra-doux pour bébé

**Formule**

| | | |
|---|---|---|
| A | Composition (C_{1B}) | 10,00% |
| | AMISOFT™CS-11 | 4,00% |
| | Parfum/Fragrance | 0,10% |
| | SEPICIDE™HB | 0,30% |
| | SEPICIDE™CI | 0,20% |
| | Eau | Q.S. 100,00% |
| B | Eau | 20,00% |
| | CAPIGEL™ 98 | 3,50% |
| | Tromethamine | Q.S. pH = 7,2 |

Mode opératoire : Mélanger tous les ingrédients de la phase A dans l'ordre indiqué jusqu'à l'obtention d'une phase A limpide. De façon séparée, ajouter le CAPIGEL™98 dans l'eau, puis ajouter cette phase B ainsi préparée dans la phase A et ajuster le pH à 7,2 à l'aide de la trométhamine.

### B.7 Lait de toilette pour bébé

**Formule**

| | | |
|---|---|---|
| A | SIMULSOL™165 | 2,00% |
| | MONTANOV™202 | 1,00% |
| | LANOL™99 | 3,00% |
| | Dimethicone | 1,00% |
| | Isohexadecane | 3,00% |
| B | Eau | Q.S. 100,00% |
| C | SEPIPLUS™400 | 0,30% |
| D | Composition (C_{1A}) | 6,35% |
| E | SEPICIDE™HB | 0,30% |
| | DMDM Hydantoin | 0,20% |
| | Parfum/Fragrance | 0,10% |

Mode opératoire : Faire chauffer séparément les phases A et B constituées par mélange des différents constituants. Ajouter la phase C dans la phase grasse chaude et réaliser l'émulsion en versant la phase aqueuse ; homogénéiser quelques minutes sous forte agitation (par l'intermédiaire d'une turbine rotor/stator). Puis ajouter la phase D dans l'émulsion chaude et refroidir l'émulsion sous agitation modérée jusqu'à retour à température ambiante. Ajouter la phase E à 40°C.

### B.8 Lotion poudrée nettoyante pour peaux sensibles

**Formule**

| | | |
|---|---|---|
| A | LIPACIDE™C8G | 0,95% |
| | Méthyl paraben | 0,10% |
| | Ethyl paraben | 0,024% |
| | Propyl paraben | 0,0119% |
| | Butyl paraben | 0,024% |
| | Isobutyl paraben | 0,0119% |
| | Eau | 20,00% |
| | Disodium EDTA | 0,10% |
| | Triethanolamine | 1,38% |
| B | Composition (C_{1B}) | 1,80% |
| | Parfum/Fragrance | 0,10% |
| C | SEPICALM™S | 0,28% |
| | Eau | Q.S. 100,00% |
| | Acide lactique | Q.S. pH = 5,2 |
| D | MICROPEARL™M310 | 5,00% |

Mode opératoire : Solubiliser les ingrédients de la phase A dans l'eau à 80°C. Solubiliser séparément le parfum dans la composition (E₄) pour préparer la phase B. Ajouter la phase A refroidie sur la phase B, puis introduire le SEPICALM™S et le complément d'eau. Vérifier le pH final et optionnellement l'ajuster à environ 5,2. Ajouter alors le MICROPEARL™ M310.

### B.9 Gel douche enfants

**Formule**

| | | |
|---|---|---|
| A | Eau | 56,06% |
| | SEPIMAX™Zen | 3,00% |
| | SEPIPLUS™S | 0,80% |
| B | PROTEOL™OAT | 20,80% |
| | ORAMIX™NS 10 | 9,30% |
| | AMONYL™265 BA | 5,10% |
| C | Composition (C_{1C}) | 2,00% |
| | Glycéryl Glucoside | 1,00% |
| | Phenoxyéthanol & Ethylhexyl Glycérine | 1,00% |
| | Parfum/Fragrance | 0,90% |
| | Colorant | 0,04% |

Mode opératoire : disperser le SEPIMAX™ZEN dans l'eau et agiter à l'aide d'un agitateur mécanique muni d'une défloculeuse, d'une contrehélice et d'une pâle de type ancre, jusqu'à l'obtention d'un gel parfaitement lisse. Ajouter le SEPIPLUS™S puis agiter jusqu'à ce que le mélange soit homogène. Ajouter ensuite les ingrédients de la phase B, homogéniser et ajouter individuellement les additifs de la phase C. Ajuster le pH à 6.0 - 6.5.

### B.10 BB Crème

**Formule**

| | | |
|---|---|---|
| A | EASYNOV™ | 2,30% |
| | LANOL™ 99 | 1,00% |
| | SEPIMAT™ H10W | 1,00% |
| | Ethylhexyl methoxycinnamate | 5,00% |
| B | Cyclométhicone | 6,00% |
| | Triethoxycaprylsilane & Alumina-silane & Titanium Oxide | 8,00% |
| | Iron Oxide red & Triethoxycaprylsilane | 0,24% |
| | Iron Oxide yellow & Triethoxycaprylsilane | 0,66% |
| | Iron Oxide black & Triethoxycaprylsilane | 0,09% |
| | Parfum/Fragrance | 0,10% |
| C | Eau | qs 100% |
| | SEPINOV™EMT10 | 1,20% |
| D | Composition (C_{1D}) | 2,00% |
| | SEPITONIC™M3 | 1,00% |
| | Phenoxyéthanol & Ethylhexyl Glycérine | 1,00% |

Mode opératoire : Préparer la phase B par mélange des différents ingrédients et homogénéiser à l'aide d'un mélangeur muni d'un système de rotor-sator à une vitesse de rotation de 4500 tours par minute, pendant une durée de 6 minutes. Préparer la phase C par addition du SEPINOV™EMT10 sur le mélange d'eau et de glycérol et homogénéiser à l'aide d'un mélangeur muni d'un système de rotor-sator à une vitesse de rotation de 4000 tours par minute pendant 4 minutes. Ajouter les phases A et B sur la phase C, et agiter le mélange résultant à l'aide d'un agitateur mécanique muni d'un pâle de type ancre, à une vitesse de 30 tours par minute pendant 2 minutes, puis à une vitesse de 50 tours par minute pendant 20 minutes. Ajouter un à un les composants de la phase 5 et agiter à une vitesse de 50 tours par minute pendant 25 minutes.

### B.11 Spray Solaire haute Protection SPH supérieur à 30

**Formule**

| | | |
|---|---|---|
| A | MONTANOV™L | 1,00% |
| | MONTANOV™82 | 1,00% |
| | C12-15 Alkylbenzoate | 17,00% |
| | Dimethicone | 3,00% |
| | Octocrylène | 6,00% |
| | Ethylhexyl methoxycinnamate | 6,00% |
| | Bis-ethylhexyloxyphenol Méthoxypenyl Triazine | 3,00% |
| | Tocophérol | 0,05% |
| B | Eau | qs 100% |
| C | SIMULGEL™INS 100 | 0,50% |
| | Cyclodiméthicone | 5,00% |
| D | Composition (C_{1C}) | 3,00% |
| | Phenoxyéthanol & Ethylhexyl Glycérine | 1,00% |
| | Parfum/Fragrance | 0,20% |
| E | Methylene Bis-Benzotriazolyl | |
| | Tetraméthylbutylphénol | 10,00% |
| | Acide citrique 25% | qs pH = 5 |

SEPICALM™S : Mélange de N-cocoyl aminoacides, de sarcosine, d'aspartate de potassium et d'aspartate de magnésium tel que décrit dans WO 98/09611 .
PROTEOL™APL : Mélange de sels de sodium de N-cocoyl aminoacides, obtenus par acylation des acides aminés caractéristiques du jus de pomme ;
SEPICIDE™HB : Mélange de phénoxyéthanol, de méthylparaben, d'éthylparaben, de propylparaben et de butylparaben, est un agent conservateur.
CAPIGEL™98 : Copolymère d'acrylates ;
SEPICIDE™CI : Imidazoline urée, est un agent conservateur ;
SEPICIDE™HB : Mélange de phénoxyéthanol, de méthylparaben, d'éthylparaben, de propylparaben, de butylparaben et d'isobutylparaben, est un agent conservateur ;
SEPICALM™VG : Mélange de N-palmitoyl proline sous forme de sel de sodium et d'extrait de fleur de Nymphéa Alba ;
EUXYL™PE9010 : Mélange de phénoxyéthanol et d'ethyl hexyl glycérine ;
PROTEOL™OAT : Mélange de N-lauryl aminoacides obtenus par hydrolyse totale de protéine d'avoine tel que décrit dans WO 94/26694 ;
MONTALINE™C40 : Sel de chlorure de Cocamidopropyl betaïnamide de Monoéthanolamine.
AMISOFT™CS-11 : Sel de sodium de N-cocoyl glutamate ;
SIMULSOL™165 : Mélange de stéarate de PEG-100 et de stéarate de glycérol ;
MONTANOV™202 (alcool arachydilique, alcool béhénique et arachidyl glucoside), est une composition auto-émulsionnable telle que celles décrites dans EP 0 977 626 ;
LANOL™99 : Isononoate d'isononyle ;
SEPIPLUS™400 : Latex inverse auto-inversible de polyacrylates dans le polyisobutene et comportant du polysorbate 20, tel que décrit dans WO2005/040230 ;
LIPACIDE™C8G : Capryloyl glycine commercialisé par la société SEPPIC ;
MICROPEARL : Polymère polyméthylméthacrylate réticulé se présentant sous forme de poudre et utilisé comme modificateur de texture ;
SEPIMAX™Zen (nom INCI : Polyacrylate Crosspolymer-6) : Polymère épaississant se présentant sous la forme d'une poudre ;
SEPIPLUS™S (nom INCI : Hydroxyethyl Acrylate / Sodium Acryloyldimethyl Taurate Copolymer & Polyisobutene & PEG-7 Trimethylolpropane Cononut Ether) : Latex inverse auto-inversible ;
AMONYL™265 BA (nom INCI : Cocobétaïne) : Agent tensioactif amphotère moussant ;
SEPINOV™EMT10 (nom INCI : Hydroxyethyl Acrylate / Sodium Acryloyldimethyl Taurate Copolymer) : Copolymère épaississant se présentant sous la forme d'une poudre ;
EASYNOV™ (nom INCI : Octyldodecanol and Octyldodecyl Xyloside and PEG-30 Dipolyhydroxystearate) : Agent émulsionnant à tendance lipophile ;
SEPIMAT™H10 FW (nom INCI :Methyl Methacrylate Crosspolymer and Squalane) : Ppolymère utilisé comme agent de texture ;
SEPITONIC™M3 (nom INCI : Magnesium Aspartate and Zinc Gluconate and Copper Gluconate) : Mélange utilisé comme agent antiradicalaire et énergisant pour les cellules ;
MONTANOV™L (nom INCI : C14-22 Alcohols and C12-20 Alkylglucoside) : Agent émulsionnant ;
MONTANOV™82 (nom INCI : Cetearyl Alcohol and Coco-glucoside) : Agent émulsionnant ;
SIMULGEL™INS100 (nom INCI :Hydroxyethyl Acrylate/Sodium Acryloydimethyl Taurate Copolymer and isohexadecane and Polysorbate 60) : Agent épaississant polymèrique ;

## Revendications

1. Composition (C₁) comprenant pour 100% de sa masse :
(1)- Une quantité supérieure à 0% en masse et inférieure ou égale à 50% en masse d'un polyol de formule (I) :
HO-CH₂-CHOH-CH₂-OH (I) ;
(2)- De 25% en masse à 95% en masse d'une composition (C_{R}) représentée par la formule (II) :
HO-CH₂-CHOH-CH₂-O-(Rham)ₓ-H (II),
formule (II) dans laquelle Rham représente le reste du rhamnose et x, qui indique le degré moyen de polymérisation dudit reste Rham, représente un nombre décimal supérieur à 1,0 et inférieur ou égal à 2.5,
(3)- Une quantité supérieure à 0% en masse et inférieure ou égale à 20% en masse de rhamnose,

2. Composition (C₁) telle que définie à la revendication 1, comprenant pour 100% de sa masse :
(1)- de 15% à 45% masse de glycérol ;
(2)- de 30% en masse à 70% en masse de la (C_{R}) représentée par la formule (II) telle que définie précédemment ;
(3)- de 5% à 20% en masse de rhamnose.

3. Composition C₁ telle que définie à la revendication 1, comprenant pour 100% de sa masse :
(1)- de 5% à 15% en masse de glycérol ;
(2)- de 65% en masse à 85% en masse de la (C_{R}) représentée par la formule (II) telle que définie précédemment ;
(3)- de 3% à 10% en masse de rhamnose.

4. Procédé de préparation de la composition (C₁) telle que définie à l'une quelconque des revendications 1 à 3, **caractérisé en ce qu'**il comprend les étapes successives suivantes :
Une **étape a)** de chauffage sous agitation douce du polyol de formule (I) telle que définie précédemment, jusqu'à le porter à une température supérieure (T₁) d'au moins 5°C au-dessus de sa température de fusion ;
Une **étape b)** de dispersion et d'homogénéisation sous agitation de rhamnose monohydraté[1] dans le milieu précédemment fondu dans le rapport stoechiométrique souhaité ;
Une **étape c)** d'acétalisation en ajoutant sous agitation dans le milieu liquide issu de l'étape b) une quantité catalytique d'acide fort, en maintenant le mélange réactionnel tout sous vide partiel et en distillant l'eau formée ; et, si nécessaire ou si désiré,
Une **étape d)** de neutralisation de la solution obtenue à l'issue de l'étape d), pour obtenir ladite composition (C₁).

5. Composition cosmétique ou dermocosmétique topique (C), **caractérisée en ce qu'**elle comprend une quantité efficace de la composition (C₁) telle que définie à l'une quelconque des revendications 1 à 3.

6. Composition pharmaceutique ou dermopharmaceutique topique (C'), **caractérisée en ce qu'**elle comprend une quantité efficace de la composition (C₁) telle que définie à l'une quelconque des revendications 1 à 3.

## Patentansprüche

1. Zusammensetzung (C₁), umfassend pro 100 % ihrer Masse:
(1) - eine Menge von mehr als 0 Massen-% und weniger als oder gleich 50 Massen-% eines Polyols der Formel (I):
**HO-CH₂-CHOH-CH₂-OH** **(I) ;**
(2) - 25 Massen-% bis 95 Massen-% einer Zusammensetzung (C_{R}), dargestellt durch die Formel (II):
**HO-CH₂-CHOH-CH₂-O-(Rham)ₓ-H** (II),
Formel (II), worin Rham den Rhamnoserest darstellt und x, das den durchschnittlichen Polymerisationsgrad des Rham-Restes angibt, eine Dezimalzahl größer als 1,0 und kleiner als oder gleich 2,5 darstellt;
(3) - eine Menge von mehr als 0 Massen-% und weniger als oder gleich 20 Massen-% Rhamnose.

2. Zusammensetzung (C₁) nach Anspruch 1, umfassend pro 100 % ihrer Masse:
(1) - 15 bis 45 Massen-% Glycerin;
(2) - 30 Massen-% bis 70 Massen-% der durch die vorstehende Formel (II) dargestellten (C_{R});
(3) - 5 bis 20 Massen-% Rhamnose.

3. Zusammensetzung C₁ nach Anspruch 1, umfassend pro 100 % ihrer Masse:
(1) - 5 bis 15 Massen-% Glycerin;
(2) - 65 Massen-% bis 85 Massen-% der durch die vorstehende Formel (II) dargestellten (C_{R});
(3) - 3 bis 10 Massen-% Rhamnose.

4. Verfahren zur Herstellung der Zusammensetzung (C₁) nach einem der Ansprüche 1 bis 3, **dadurch gekennzeichnet, dass** es die folgenden aufeinanderfolgenden Schritte umfasst:
einen **Schritt a)** des Erhitzens unter leichtem Rühren des Polyols der Formel (I), wie vorstehend definiert, bis es auf eine höhere Temperatur (T₁) von mindestens 5°C oberhalb seines Schmelzpunkts gebracht wird;
einen **Schritt b)** der Dispersion und Homogenisation unter Rühren von Rhamnosemonohydrat[1] in dem zuvor geschmolzenen Medium im gewünschten stöchiometrischen Verhältnis;
einen **Schritt c)** der Acetalisierung durch Zugabe einer katalytischen Menge einer starken Säure unter Rühren zu dem aus Schritt b) erhaltenen flüssigen Medium, wobei das gesamte Reaktionsgemisch unter Teilvakuum gehalten und das gebildete Wasser abdestilliert wird; und, falls notwendig oder gewünscht,
einen **Schritt d)** der Neutralisation der aus Schritt d) erhaltenen Lösung, um die Zusammensetzung (C₁) zu erhalten.

5. Kosmetische oder dermokosmetische topische Zusammensetzung (C), **dadurch gekennzeichnet, dass** sie eine wirksame Menge der Zusammensetzung (C₁) nach einem der Ansprüche 1 bis 3 umfasst.

6. Pharmazeutische oder dermopharmazeutische topische Zusammensetzung (C'), **dadurch gekennzeichnet, dass** sie eine wirksame Menge der Zusammensetzung (C₁) nach einem der Ansprüche 1 bis 3 umfasst.

## Claims

1. Composition (C₁) comprising, per 100% of its mass:
(1) - an amount of greater than 0% by mass and less than or equal to 50% by mass of a polyol of formula (I):
HO-CH₂-CHOH-CH₂-OH (I);
(2)- from 25% by mass to 95% by mass of a composition (C_{R}) represented by formula (II):
HO-CH₂-CHOH-CH₂-O- (Rham) ₓ-H (II),
in which formula (II) Rham represents the rhamnose residue and x, which indicates the average degree of polymerization of said Rham residue, represents a decimal number greater than 1.0 and less than or equal to 2.5;
(3) - an amount of greater than 0% by mass and less than or equal to 20% by mass of rhamnose.

2. Composition (C₁) as defined in Claim 1, comprising, per 100% of its mass:
(1) - from 15% to 45% by mass of glycerol;
(2) - from 30% by mass to 70% by mass of the (C_{R}) represented by the formula (II) as defined previously;
(3) - from 5% to 20% by mass of rhamnose.

3. Composition C₁ as defined in Claim 1, comprising, per 100% of its mass:
(1) - from 5% to 15% by mass of glycerol;
(2) - from 65% by mass to 85% by mass of the (C_{R}) represented by the formula (II) as defined previously;
(3) - from 3% to 10% by mass of rhamnose.

4. Process for preparing the composition (C₁) as defined in any one of Claims 1 to 3, **characterized in that** it comprises the following successive steps:
a **step a)** of heating, with gentle stirring, the polyol of formula (I) as defined previously, until it is brought to a higher temperature (T₁) of at least 5°C above its melting point;
a **step b)** of dispersing and homogenizing, with stirring, rhamnose monohydrate[1] in the previously molten medium in the desired stoichiometric ratio;
a **step c)** of acetalization by adding, with stirring, to the liquid medium obtained from step b), a catalytic amount of strong acid, while maintaining all the reaction mixture under partial vacuum and distilling off the water formed; and, if necessary or if desired,
a **step d)** of neutralizing the solution obtained on conclusion of step d), to obtain said composition (C₁).

5. Topical cosmetic or dermocosmetic composition (C), **characterized in that** it comprises an effective amount of composition (C₁) as defined in any one of Claims 1 to 3.

6. Topical pharmaceutical or dermopharmaceutical composition (C'), **characterized in that** it comprises an effective amount of composition (C₁) as defined in any one of Claims 1 to 3.
